# EUROPEAN PATENT APPLICATION

(11) **EP 2 851 063 A1**
(43) Date of publication of application: **25.03.2015**
(21) Application number: 13185181.8
(22) Date of filing: 19.09.2013
(51) Int. Cl.: A61K 9/00, A61K 9/51, A61K 31/197

(54) **Gabapentin compositions for treating or preventing ocular pain**

(71) Applicant: Consorzio Universitario Unifarm, 95125 Catania (IT)
(72) Inventor: Bucolo, Claudio, 95125 CATANIA (IT); Drago, Filippo, 95125 CATANIA (IT); Pignatello, Rosario, 95125 CATANIA (IT)
(74) Representative: Minoja, Fabrizio

(57) **Abstract**

The present invention relates to a topical ophthalmic formulation for use in the treatment of ocular pain comprising gabapentin or a salt thereof and nanoparticles of copolymers of polyethylacrylate, methyl-methacrylate and chlorotrimethyl-ammonioethylmethacrylate having a content of quaternary ammonium groups between 4.5 and 12%.

## Description

### FIELD OF THE INVENTION

This invention relates to a topical ophthalmic formulation comprising gabapentin and a polymer-based nanocarrier for treating or preventing ocular pain.

### DESCRIPTION OF THE RELATED ART

Pain conditions involving the eye can arise in numerous instances, such as herpes, surgical procedures and post- surgical recovery, dry eye syndrome, foreign body stimulus, inflammation, accidental trauma, neuropathic corneal pain, diabetes. For example, ocular pain can result from photorefractive keratotomy ("PRK"), a vision correcting, surgical procedure whereby a laser is used to shape the cornea. This process involves the photoablation of Bowman's membrane and the stromal levels of the cornea. As a result, the denuding of the nerve-containing epithelial layers of the cornea can cause some patients to experience pain following laser surgery until the epithelium regenerates. Another example is the post-herpetic neuralgia, emergency department care of herpes zoster ophthalmicus (HZO) includes local wound care, adequate analgesia, starting antiviral agents, and antibiotics for secondary bacterial infection.

Gabapentin [(1-(aminomethyl)cyclohexane acetic acid)] is an antiepileptic drug, active in various animal models of epilepsy, and effective in decreasing the frequency of seizures in patients. Gabapentin, although is a [gamma]-aminobutyric acid (GABA) structural analogue, does not significantly bind to GABA receptors, but binds to the [alpha]2[delta] subunit of a calcium channel. In recent years gabapentin has received much attention as an analgesic after a wide variety of ophthalmic and non-ophthalmic surgical procedures but with mixed results. Many ophthalmologists adapted the use of oral gabapentin for refractive surgery based on anecdotal word-of-mouth reports of its usefulness.

Patent EP 2316420 discloses the topical use of gabapentin formulations for treating ocular pain and/or ocular inflammation.

It was now surprisingly found that polymeric nanoparticle formulations made of Eudragit^{®} Retard polymers and containing gabapentin exhibited an improved pharmacological profile including a significantly better activity and a prolonged effect in reducing ocular pain.

In previous studies the production of nanosuspensions of Eudragit^{®} Retard matrices have been described:
Pignatello R, Amico D, Chiechio S, Giunchedi P, Spadaro C, Puglisi G. Preparation and analgesic activity of Eudragit RS100 microparticles containing diflunisal. Drug Delivery 2001;8:35-45.

Pignatello R, Bucolo C, Spedalieri G, Maltese A, Puglisi G. Flurbiprofen-loaded acrylate polymer nanosuspensions for ophthalmic application. Biomaterials. 2002a;23(15):3247-55.

Pignatello R, Bucolo C, Ferrara P, Maltese A, Puleo A, Puglisi G. Eudragit RS100 nanosuspensions for the ophthalmic controlled delivery of ibuprofen. Eur J Pharm Sci. 2002b;16(1-2):53-61.

Bucolo C, Maltese A, Puglisi G, Pignatello R.Enhanced ocular anti-inflammatory activity of ibuprofen carried by an Eudragit RS100 nanoparticle suspension. Ophthalmic Res. 2002;34(5):319-23.

Pignatello R, Ricupero N, Bucolo C, Maugeri F, Maltese A, Puglisi G. Preparation and characterization of eudragit retard nanosuspensions for the ocular delivery of cloricromene. AAPS PharmSciTech. 2006;7(1):E27.

These nanosystems were prepared using an adaptation of a solvent evaporation technique called "Quasi-emulsion solvent diffusion" (QESD), a production method that allows to use highly biocompatible solvents, such as ethanol, and use of low temperatures, being suitable also for thermosensitive active compounds. In those papers, Eudragit^{®} Retard nanoparticle suspensions have been loaded with some non-steroidal anti-inflammatory agents (NSAIDs), such as ibuprofen, flurbiprofen, and cloricromene. Other authors applied the QESD technique for the production of Eudragit nanoparticles, encapsulating other NSAIDs or other drugs such as antiinflammatory steroids, amphotericin B, sulfacetamide, or insulin:
Adibkia K, Javadzadeh Y, Dastmalchi S, Mohammadi G, Niri FK, Alaei-Beirami M. Naproxen-eudragit RS100 nanoparticles: preparation and physicochemical characterization. Colloids Surf B Biointerfaces. 2011;83(1):155-9.
Adibkia K, Siahi Shadbad MR, Nokhodchi A, Javadzedeh A, Barzegar-Jalali M, Barar J, Mohammadi G, Omidi Y. Piroxicam nanoparticles for ocular delivery: physicochemical characterization and implementation in endotoxin-induced uveitis. J Drug Target. 2007;15, 407-16.

Das S, Suresh PK. Nanosuspension: a new vehicle for the improvement of the delivery of drugs to the ocular surface. Application to amphotericin B. Nanomedicine. 2011;7(2):242-7.

Elshafeey AH, Kamel AO, Awad GA. Ammonium methacrylate units polymer content and their effect on acyclovir colloidal nanoparticles properties and bioavailability in human volunteers. Colloids Surf B Biointerfaces. 2010, 75(2):398-404.

Mandal B, Alexander KS, Riga AT. Sulfacetamide loaded Eudragit® RL100 nanosuspension with potential for ocular delivery. J Pharm Pharm Sci. 2010;13(4):510-23).

The above examples all refer to the encapsulation of active molecules with lipophilic properties (most having an aromatic structure) and a molecular weight higher than 300 Da. In fact, the original works by Pignatello et al. (see above) were based on the occurring of electrostatic interactions between acidic drugs, such as NSAIDs, and the positively charged Eudragit^{®} Retard backbones (because of the presence of the alkylammonium groups). This strategy has been followed by some of the other Authors who prepared analogous nanoparticle systems.

Furthermore, since the method of preparation (both the used QESD technique as well as most of known nanoparticle production methods) involves the presence of a large aqueous phase, the encapsulation of small, polar and water soluble compounds, such as gabapentin, is difficult to foresee and a large escaping of the active compound in the external aqueous phase is to be expected. Surprisingly, we found that, by adapting in a suitable and original way the working conditions, gabapentin can be efficiently encapsulated and retained in Eudragit^{®} Retard nanomatrices as well as other polymer- or lipid-based nanosystems (see below).

The proposed invention instead represents the first example of a nanoparticle suspension, based on Eudragit^{®} Retard matrices, and obtained by the QESD technique which envisages the incorporation of a small molecule (MW = 171.237), a non-aromatic structure and with a high hydrophilic character (LogP = -1.10; LogD = -1.44).

Furthermore the present invention provide a method for producing of very small volumes of nanosuspension (in the order of 2-5 ml) and with very small amount of active ingredient (tens or hundreds of micrograms), i.e. much lower than those used in the works cited above and suitable for the encapsulation of very powerful, and/or expensive and/or available in limited quantity active compounds.

### DESCRIPTION OF THE INVENTION

The present invention relates to topical ophthalmic nanoparticle formulations comprising gabapentin or a salt thereof and copolymers of polyethylacrylate, methyl-methacrylate and chlorotrimethyl-ammonioethylmethacrylate having a content of quaternary ammonium groups between 4.5-12% for use in the treatment of ocular pain. Examples of copolymers of polyethylacrylate, methyl-methacrylate and chlorotrimethyl-ammonioethylmethacrylate are Eudragit polymers such as Eudragit RS100 (RS) and RL100 (RL) which contain an amount of quaternary ammonium groups between 4.5-6.8% and 8.8-12%, respectively for RS and RL.

Ocular pain may be of various origin such as, but not limited to these, post-herpetic neuralgia, post-surgical procedures, dry eye syndrome, diabetes. The formulations are administered by instillation in the eye. The formulations of the invention improve the bioavailability and increase the ocular absorption of gabapentin. They extend the effects of gabapentin in comparison to other gabapentin formulations and allow for a decrease in required daily dosage. In particular the formulation of the invention reduce ocular pain associated with ophthalmic surgery such as pain associated with PRK surgery.

Said copolymers are insoluble in water at physiological pH values and are capable of swelling.

Preferably, the aqueous ophthalmic nanosuspensions of the present invention comprise nanoparticles with a mean particle size from 50 to 300 nm, more preferably from 75 to 150 nm.

The copolymer nanosuspensions were prepared by means of an opportunely modified solvent evaporation method, as described in the examples, to achieve particles with dimensional and stability characteristics compatible with the ocular application. The copolymer and concentration is selected to provide a prolonged corneal residence time and an increased absorption of gabapentin in the cornea, as compared to a comparative gabapentin solution. More preferably, the aqueous ophthalmic nanosuspension of the present invention is based on Eudragit RL100 or Eudragit RS100 resins, or a mixture of them at different weight ratios. More preferably, the aqueous ophthalmic nanosuspension of the present invention has a concentration of copolymer from about 0.5 to about 10 percent, by volume, and most preferably of 5% w/v.

The concentration of gabapentin, a salt thereof, or an ester thereof in the pharmaceutical compositions of the present invention can be in the range from about 0.0001 to about 100 mg/ml (or, alternatively, from about from about 0.001 to about 50 mg/ml, or from about 0.001 to about 30 mg/ml, or from about 0.001 to about 25 mg/ml, or from about 0.001 to about 10 mg/ml, or from about 0.001 to about 5 mg/ml, or from about 0.01 to about 30 mg/ml, or from about 0.01 to about 25 mg/ml, or from about 0.01 to about 10 mg/ml, or from about 0.1 to about 10 mg/ml, or from about 0.1 to about 5 mg/ml). More preferably, the aqueous ophthalmic nanocarriers of this invention comprise an effective amount of gabapentin of 0.1 percent by volume.

The ratio between gabapentin and the polymers used to obtain the nanocarriers, is comprised in the final nanosuspension within 0.1 and 20% (w/w), more preferably in a range between 0.1 and 10% (w/w), and even more preferably in a range between 0.5 and 2% (w/w).

The compositions administered according to the present invention may also include various other ingredients, including but not limited to surfactants, tonicity agents, buffers, preservatives, co-solvents, humectants and viscosity building agents.

Various tonicity agents may be employed to adjust the tonicity of the composition, preferably to that of natural tears for ophthalmic compositions. For example, sodium chloride, potassium chloride, magnesium chloride, calcium chloride, dextrose and/or mannitol may be added to the composition to approximate physiological tonicity. Such an amount of tonicity agent will vary, depending on the particular agent to be added. In general, however, the compositions will have a tonicity agent in an amount sufficient to cause the final composition to have an ophthalmically acceptable osmolality (generally about 150-450 mOsm, preferably 250-350 mOsm).

An appropriate buffer system (e.g., sodium phosphate, sodium acetate, sodium citrate, sodium borate or boric acid) may be added to the compositions to prevent pH drift under storage conditions. The particular concentration will vary, depending on the agent employed. Preferably, however, the buffer will be chosen to maintain a target pH within the range of pH 6.0 - 7.5, which is comfortable when topically applied to the eye of a patient.

Unit dose compositions of the present invention will be sterile. The ophthalmic compositions of the present invention may be provided preservative free and packaged in unit dose form. Alternatively, the topical ophthalmic products may also be packaged in multidose form. Preservatives may thus be required to prevent microbial contamination during use. Suitable preservatives include: chlorobutanol, methyl paraben, propyl paraben, phenylethyl alcohol, disodium edetate, sorbic acid, polyquaternium-1, or other agents known to those skilled in the art. These agents may be present in individual amounts of from about 0.001 to about 5% by weight (preferably, about 0.01 % to about 2% by weight).

Suitable surfactants may be non-ionic surfactants, such as polysorbates (such as polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of Tween® 80, Tween® 60, and Tween® 20), poloxamers (synthetic block polymers of ethylene oxide and propylene oxide, such as those commonly known by their trade names of Pluronic®; e.g., Pluronic® F127 or Pluronic® F108)), or poloxamines (synthetic block polymers of ethylene oxide and propylene oxide attached to ethylene diamine, such as those commonly known by their trade names of Tetronic®; e.g., Tetronic® 1508 or Tetronic® 908, etc., other nonionic surfactants such as Brij®, Myrj®, and long chain fatty alcohols (i.e., oleyl alcohol, stearyl alcohol, myristyl alcohol, docosohexanoyl alcohol, etc.) with carbon chains having about 12 or more carbon atoms (e.g., such as from about 12 to about 24 carbon atoms). Such compounds are delineated in Martindale, 34th ed., pp. 1411-1416 (Martindale, "The Complete Drug Reference," S. C. Sweetman (Ed.), Pharmaceutical Press, London, 2005) and in Remington, "The Science and Practice of Pharmacy," 21st Ed., p. 291 and the contents of chapter 22, Lippincott Williams & Wilkins, New York, 2006).

Preclinical data indicate that the new gabapentin ocular nanosuspension is more effective than a gabapentin aqueous solution not containing the drug loaded in Eudragit Retard nanoparticles.

### Preparation of the mabapentin-loaded Eudragit nanoparticle suspensions.

The Eudragit RL100 resin, or RS100 resin, or a mixture of them at different weight ratio was dissolved under slow agitation at room temperature in absolute ethanol, to produce a 0.5-10% (w/v) solution of the polymer. To 200 µl of the above ethanol solution, gabapentin was added to produce a solution containing the required percentage of drug and the resulting solution was slowly poured, during 5 minutes, using a thin Teflon tube connected to a syringe, to 2 ml of an aqueous phase (for instance, *pro injection* water or phosphate buffered saline (PBS, pH 7.4)) containing 0.1% (w/v) of a surfactant (typically, Tween 80), and kept in an iced water bath. During the addition of the organic solution, the mixture was maintained under high-speed stirring using an UltraTurrax T25 mixer (Ika, Germany) equipped with a G10 or a G8 accessory. A stirring speed between 13,000 to 24,000 rpm was used. After a 15 minutes agitation time, the suspension was kept under magnetic stirring (100 rpm) at room temperature for 8-12 hours, to allow the complete evaporation of ethanol. The resulting nanosuspension was stored in closed vials at 4 ± 1 °C until its use.

The physico-chemical characterization (pH, osmolarity, mean particle size, polydispersity index and Zeta potential values) were measured within 24 hours from the nanoparticle preparation. All physico-chemical parameters showed to be unaltered after three months of storage at 4 ± 1 °C.

### In vivo evaluation of ocular pain reduction

Sprague-Dawley rats were divided in 4 groups and treated topically with 20 µL of each tested formulation in the right eye. After the appropriate pretreatment time of about 5 minutes, 5 µL of 0.1% aqueous formalin was applied topically in the eye. Corneal sensation was measured using a Cochet-Bonnet esthesiometer. The testing began with the nylon filament extended to the maximal length (6 cm). The end of the nylon filament was touched to the cornea. If the rat blinked (positive response) the length of the filament was recorded. If the rat did not blink then the nylon filament was shortened by 0.5 cm and the test repeated until a positive response was recorded. This process was repeated for each eye three times.

Corneal sensation was determined with a Cochet-Bonnet esthesiometer. Data are presented as the mean ± S.D. (cm) for corneal sensitivity (n = 6-12).

| Formulation | Pretreatment time (min) | Corneal sensitivity (cm; mean ± S.D.) | | | | | | |
|---|---|---|---|---|---|---|---|---|
| | | 10 min | 30 min | 60 min | 120 min | 240 min | 360 min | 480 min |
| Group 1 Control + (saline) | 5 | 6.0±0.1 | 6.0±0.2 | 6.0±0.2 | 6.0±0.1 | 6.0±0.1 | 6.0±0.2 | 5.9±0.1 |
| Group 2 (gabapentin in saline) | 5 | 4.1±0.2* | 4.5±0.3* | 5.5±0.5 | 6.0±0.3 | 6.0±0.3 | 6.0±0.1 | 6.1±0.2 |
| Group 3 (gabapentin-NP **example n. 1)** | 5 | 4.0±0.2* | 3.8±0.3* | 4.0±0.2 | 4.0±0.2 | 4.0±0.2 | 4.0±0.2 | 4.2±0.3 |
| Group 4 (gabapentin-NP **example n. 2)** | 5 | 3.9±0.5* | 3.9±0.2* | 4.1±0.2* | 4.0±0.2* | 3.9±0.2* | 3.9±0.1* | 4.0±0.1* |

| | | | | | | | | |
|---|---|---|---|---|---|---|---|---|
| *p<0.01 vs. control +. Control + = rat eye treated only with 5 µL of 0.1% aqueous formalin. | | | | | | | | |

### Ocular tolerability assay.

The potential ocular irritancy and/or damaging effects of the gabapentin-loaded Eudragit nanosuspensions were evaluated according to a modified Draize test (McDonald and Shadduck, 1977), using a slit lamp (Sbisà, Florence, Italy). New Zealand albino rabbits (Charles River, Calco, Italy), weighing 1.8-2.2 kg, and with no signs of ocular inflammation or gross abnormalities. Animal procedures conformed to the Association for Research in Vision and Ophthalmology (ARVO) resolution on the use of animals in research. The eye tissue was observed after 10 min, 6 and 24 h after the last instillation in the conjunctival fornix. The formulations were instilled in the right eye of the rabbit every 30 min for 6 h (12 treatments). The congestion, swelling and discharge of the conjunctiva were graded on a scale from 0 to 3, 0 to 4 and 0 to 3, respectively. Iris hyperemia and corneal opacity were graded on a scale from 0 to 4. The nanosuspension formulation (50 µl) was topically administered in the right eye every 30 min for 6 h (twelve treatments). At the end of the treatment, three observations at 10 min, 6 h and 24 h were carried out to evaluate the ocular tissues. Topical application of the nanosuspension formulation to rabbit eyes showed no sign of toxicity or irritation to ocular tissues (data not shown).

The tested nanotechnological formulation based on gabapentin and Eudragit Retard polymers reduces the ocular pain with a significant extension in terms of duration of the activity as compared to an aqueous gabapentin formulation without Eudragit.

The foregoing description as well as the examples which follow are intended to illustrate and not limit the scope of the invention. Other aspects, advantages and modifications with the scope of the invention will be apparent to those skilled in the art to which the invention pertains.

### Example 1: Composition n. 1

The Eudragit RL100 resin was dissolved under slow agitation at room temperature in absolute ethanol, to produce a 5% (w/v) solution of the polymer. To 200 µl of the above ethanol solution, 2 mg of gabapentin were added and the resulting solution was slowly poured, during 5 minutes, using a thin Teflon tube connected to a syringe, to 2 ml of *pro injection* water containing 0.1 % (w/v) of Tween 80 and kept in an iced water bath. During the addition of the organic solution, the mixture was maintained under high-speed stirring using an UltraTurrax T25 mixer (Ika, Germany) equipped with a G10 accessory. A stirring speed between of 24,000 rpm was used. After 15 minutes of stirring, the suspension was kept under magnetic stirring (100 rpm) at room temperature for 8-12 hours. The resulting nanosuspension was stored in closed vials at 4 ± 1 °C until its use.

The above nanosuspension showed the following physico-chemical parameters: pH 6.74, osmolarity 247 mOsm/l, mean particle size: 236.22 ± 4.99 nm, polydispersity index: 0.291 ± 0.02, and Zeta potential +24.10 ± 3.32 mV).

### Example 2: Composition n. 2

The Eudragit RL100 resin was dissolved under slow agitation at room temperature in absolute ethanol, to produce a 5% (w/v) solution of the polymer. To 200 µl of the above ethanol solution, 2 mg of gabapentin were added and the resulting solution was slowly poured, during 5 minutes, using a thin Teflon tube connected to a syringe, to 2 ml of phosphate buffered saline (PBS, pH 7.4) containing 0.1% (w/v) of Tween 80 and kept in an iced water bath. During the addition of the organic solution, the mixture was maintained under high-speed stirring using an UltraTurrax T25 mixer (Ika, Germany) equipped with a G10 accessory. A stirring speed between of 24,000 rpm was used. After 15 minutes of stirring, the suspension was kept under magnetic stirring (100 rpm) at room temperature for 8-12 hours. The resulting nanosuspension was stored in closed vials at 4 ± 1 °C until its use.

The above nanosuspension showed the following physico-chemical parameters: pH 7.63, osmolarity 304 mOsm/l, mean particle size: 152.10 ± 17.7 nm, polydispersity index: 0.359 ± 0.053, and Zeta potential +20.51 ± 0.32mV).

### Example 3: Composition n. 3

The Eudragit RL100 resin was dissolved under slow agitation at room temperature in absolute ethanol, to produce a 5% (w/v) solution of the polymer. To 200 µl of the above ethanol solution, 2 mg of gabapentin were added and the resulting solution was slowly poured, during 5 minutes, using a thin Teflon tube connected to a syringe, to 2 ml of phosphate buffered saline (PBS, pH 7.4) containing 0.1% (w/v) of Tween 80 and kept in an iced water bath. During the addition of the organic solution, the mixture was maintained under high-speed stirring using an UltraTurrax T25 mixer (Ika, Germany) equipped with a G10 accessory. A stirring speed between of 20,500 rpm was used. After 15 minutes of stirring, the suspension was kept under magnetic stirring (100 rpm) at room temperature for 8-12 hours. The resulting nanosuspension was stored in closed vials at 4 ± 1 °C until its use.

The above nanosuspension showed the following physico-chemical parameters: pH 7.39, osmolarity 304 mOsm/l, mean particle size: 71.87 ± 0.78 nm, polydispersity index: 0.275 ± 0.005, and Zeta potential +24.30 ± 1.74 mV).

### Example 4: Composition n. 4

The Eudragit RS100 resin was dissolved under slow agitation at room temperature in absolute ethanol, to produce a 5% (w/v) solution of the polymer. To 200 µl of the above ethanol solution, 2 mg of gabapentin were added and the resulting solution was slowly poured, during 5 minutes, using a thin Teflon tube connected to a syringe, to 2 ml of either *pro injection* water or phosphate buffered saline (PBS, pH 7.4), containing 0.1% (w/v) of Tween 80 and kept in an iced water bath. During the addition of the organic solution, the mixture was maintained under high-speed stirring using an UltraTurrax T25 mixer (Ika, Germany) equipped with a G10 accessory. A stirring speed between of 20,500 rpm was used. After 15 minutes of stirring, the suspension was kept under magnetic stirring (100 rpm) at room temperature for 8-12 hours. The resulting nanosuspension was stored in closed vials at 4 ± 1°C until its use.

The above nanosuspensions showed the following physico-chemical parameters: production medium: water= pH 6.67, osmolarity 249 mOsm/l, mean particle size: 356.91 ± 1.99 nm, polydispersity index: 0.338 ± 0.061, and Zeta potential +38.9 ± 3.05 mV.

Production medium: PBS= pH 7.38, osmolarity 306 mOsm/l, mean particle size: 503.89 ± 15.24 nm, polydispersity index: 0.271 ± 0.042, and Zeta potential + 25.1 ± 1.26 mV.

### Example 5: Composition n. 5

Eudragit RS100 (0.5 g) and Eudragit RL100 (0.5 g) were co-dissolved under slow agitation at room temperature in 200 ml of absolute ethanol. To 200 µl of the above solution, 2 mg of gabapentin were added and the resulting solution was slowly poured, during 5 minutes, using a thin Teflon tube connected to a syringe, to 2 ml of either *pro injection* water or phosphate buffered saline (PBS, pH 7.4), both containing 0.1% (w/v) of Tween 80 and kept in an iced water bath. During the addition of the organic solution, the mixture was maintained under high-speed stirring using an UltraTurrax T25 mixer (Ika, Germany) equipped with a G8 accessory. A stirring speed between of 20,500 rpm was used. After 15 minutes of stirring, the suspension was kept under magnetic stirring (100 rpm) at room temperature for 8-12 hours. The resulting nanosuspension was stored in closed vials at 4 ± 1°C until its use.

The above nanosuspensions showed the following physico-chemical parameters:
Production medium: water= pH 6.30, osmolarity 255 mOsm/l, mean particle size: 91.70 ± 12.87 nm, polydispersity index: 0.40 ± 0.08, and Zeta potential +30.00 ± 3.32 mV).
Production medium: PBS= pH 7.37, osmolarity 306 mOsm/l, mean particle size: 243.10 ± 4.60 nm, polydispersity index: 0.509 ± 0.046, and Zeta potential + 22.9 ± 0.70 mV.

### Example 6: In vitro drug release assay

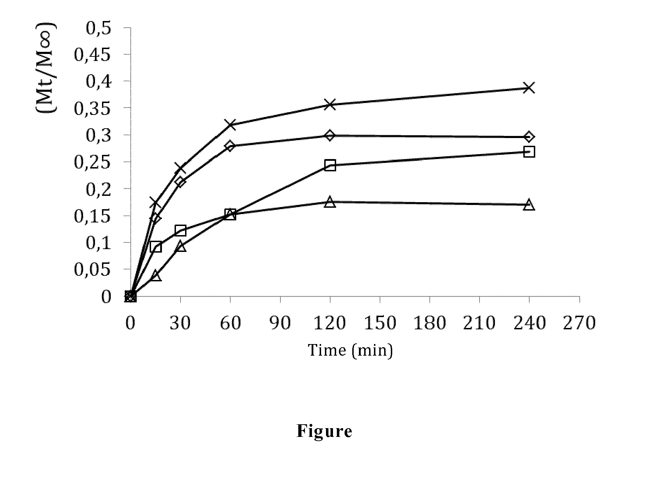

One milliliter of each nanosuspension was loaded inside a SpectraPor dialysis membrane bag (cut-off: 3,500 Da), immersed in 20 ml of phosphate buffered saline (PBS, pH 7.4), which represents the external phase, at room temperature and under a slow magnetic stirring. At specific time intervals (15, 30, 60, 120 and 240 minutes), 2 ml-aliquots of PBS were withdrawn, immediately restoring the original volume with an equivalent amount of fresh buffer. The samples were filtered through a 0.45 mm nylon syringe filter and analyzed by LC-MS/MS with a triple-quadrupole mass spectrometer (Agilent G-5610). Examples of gabapentin release from the Eudragit Retard nanosuspensions are given in Figure 1 wherein Gabapentin release profiles (dialysis method, room temperature) from different Eudragit Retard nanosuspensions are reported: Υ: composition #1, 0: composition #2, Δ: composition #4, x: composition #5.

## Claims

1. A topical ophthalmic formulation for use in the treatment of ocular pain comprising gabapentin or a salt thereof and nanoparticles of copolymers of polyethylacrylate, methyl-methacrylate and chlorotrimethyl-ammonioethylmethacrylate having a content of quaternary ammonium groups between 4.5 and 12%.

2. The topical ophthalmic formulation for use in the treatment of ocular pain according to claim 1 wherein the copolymer has a content of quaternary ammonium groups between 4.5 and 6.8% or between 8.8 and 12%.

3. The topical ophthalmic formulation for use in the treatment of ocular pain according to anyone of the preceding claims wherein the copolymer is Eudragit RS or RL.

4. The topical ophthalmic formulation for use in the treatment of ocular pain according to anyone of the preceding claims wherein the nanoparticles have a mean particle size from 50 to 300 nm.

5. The topical ophthalmic formulation for use in the treatment of ocular pain according to claim 4 wherein the nanoparticles have a mean particle size from 75 to 150 nm.

6. The topical ophthalmic formulation for use in the treatment of ocular pain according to anyone of the preceding claims wherein the copolymer has a concentration from about 0.5 to about 10% w/v.

7. The topical ophthalmic formulation for use in the treatment of ocular pain according to claim 6 wherein the copolymer has a concentration of 5% w/v.

8. The topical ophthalmic formulation for use in the treatment of ocular pain according to anyone of the preceding claims wherein the concentration of gabapentin or a salt thereof, is in the range from about 0.0001 to about 100 mg/ml.

9. The topical ophthalmic formulation for use in the treatment of ocular pain according to anyone of the preceding claims wherein the ratio between gabapentin and the copolymer is comprised within 0.1 and 20% (w/w).

10. The topical ophthalmic formulation for use in the treatment of ocular pain according to anyone of the preceding claims further containing surfactants, tonicity agents, buffers, preservatives, co-solvents, humectants or surfactants, tonicity agents, buffers, preservatives, co-solvents, humectants and viscosity building agents. viscosity building agents.

11. The topical ophthalmic formulation for use in the treatment of ocular pain according to anyone of the preceding claims wherein ocular pain is associated to post-herpetic neuralgia, post-surgical procedures, dry eye syndrome, diabetes.
